(19) Europäisches Patentamt
European Patent Office
Office européen des brevets

(11) Numéro de publication : **0 147 298**
**B1**

(12) **FASCICULE DE BREVET EUROPÉEN**

(45) Date de publication du fascicule du brevet : **26.04.89**

(51) Int. Cl.[4] : **C 07 B 45/04**, C 07 C147/06, C 07 C143/70

(21) Numéro de dépôt : **84402622.9**

(22) Date de dépôt : **18.12.84**

(54) **Procédé de sulfonylation.**

(30) Priorité : **22.12.83 FR 8320538**

(43) Date de publication de la demande : **03.07.85 Bulletin 85/27**

(45) Mention de la délivrance du brevet : **26.04.89 Bulletin 89/17**

(84) Etats contractants désignés : **AT BE CH DE FR GB IT LI LU NL SE**

(56) Documents cités :
**EP-A- 0 084 743**
**EP-A- 0 084 744**
**GB-A- 1 351 453**
**Finar-organic chemistry, pp. 689,692**
**Le dossier contient des informations techniques présentées postérieurement au dépôt de la demande et ne figurant pas dans le présent fascicule.**

(73) Titulaire : **RHONE-POULENC CHIMIE**
**25, quai Paul Doumer**
**F-92408 Courbevoie Cedex (FR)**

(72) Inventeur : **Desbois, Michel**
**526, Chemin du Bois**
**F-69140 - Rillieux (FR)**

(74) Mandataire : **Le Pennec, Magali et al**
**RHONE-POULENC INTERSERVICES Service Brevets Chimie 25, Quai Paul Doumer**
**F-92408 Courbevoie Cedex (FR)**

**EP 0 147 298 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de sulfonylation de dérivés aromatiques.

Elle concerne plus particulièrement un procédé de sulfonylation de dérivés aromatiques désactivés.

On entend au sens de la présente invention par dérivé aromatique désactivé, tout dérivé aromatique porteur d'au moins un groupe $-AC_n X_1(X_2)_n (X_3)_n$ où A représente une liaison covalente, un atome d'oxygène ou de soufre, $X_1$, $X_2$, $X_3$ représentent chacun un halogène identique ou différent et où n est égal à 0 ou 1.

On connaît dans l'art antérieur, des documents décrivant des procédés de sulfonylation.

Ces documents peuvent être regroupés en trois catégories.

La première catégorie de documents (Chemical Abstract 1941, 35, 2123 ; brevet français N° 2 381 027) divulgue des procédés de sulfonylation par l'acide sulfurique en milieu HF à haute température (140 à 150 °C) sur des dérivés activés. Ces procédés ne s'appliquent jamais aux dérivés désactivés.

La deuxième catégorie de documents (brevet polonais N° 102 679, brevet anglais N° 1 351 453, demande de brevet allemand 2 252 571-GBA 1351 453) divulgue des procédés de sulfonylation par $H_2SO_4$ seul à très haute température (230 à 250 °C), ou à très haute température et pression élevée.

La troisième catégorie de documents (Shestov and N.A. Osipova Sb Statei Nauchn-Issled Inst. Organ. Poluprod. i Krasitelei 1961, No 2, 13-45 et demande de brevet allemand 1 950 394) divulgue des procédés de sulfonylation par $H_2SO_4$ en présence de catalyseur tel que $Na_2SO_4$ ou l'hexachloroéthane à haute température (150° environ).

Dans cette dernière catégorie, on peut aussi inclure un procédé de sulfonylation décrit par Kozlov et ses collaborateurs dans Zh. Org. Khim. 1978, 14 (9), 1918-21 qui divulgue une sulfonylation en présence d'un catalyseur à base de pentafluorure d'antimoine dans l'acide fluorhydrique à 0 °C. Cette sulfonylation est réalisée sur des dérivés aromatiques activés. EPA 84743 et EPA 84744 décrivent des méthodes de sulfonylation ne mettant pas en œuvre l'acide sulfurique comme agent de sulfonylation.

Aucun des procédés préalablement cités ne permet de réaliser une sulfonylation sur des dérivés aromatiques désactivés à une température inférieure à 120 °C.

Il a maintenant été découvert un procédé de sulfonylation permettant notamment d'atteindre cet objectif, depuis longtemps recherché.

En effet, la présente invention concerne un procédé de préparation de diarylsulfones de formule (III)

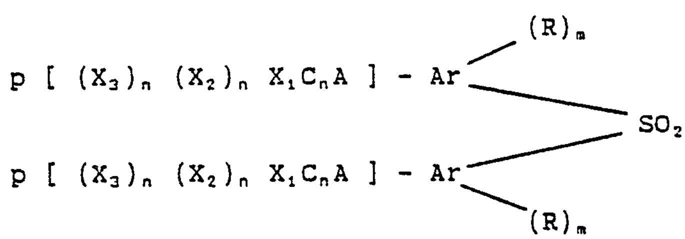

(III)

dans laquelle :

Ar représente un radical aromatique mono ou polycyclique

A représente une liaison covalente, un atome d'oxygène ou de soufre

$X_1$, $X_2$ et $X_3$ représentent chacun un atome d'halogène identique ou différent

n est compris entre 0 et 2 et quand A est une liaison covalente

n est égal à 0

p est égal à 1 ou 2

R est choisi parmi l'hydrogène, un groupe halogéno, alkyle de C1 à C6, alkoxy, alkylthio, hydroxyle

m est égal à 1 ou 2

caractérisé par le fait que l'on fait réagir un composé de formule $(R)_m-Ar-[AC_n X_1(X_2)_n(X_3)_n]$ p dans laquelle R, Ar, A, $X_1$, $X_2$, $X_3$, m, n, p ont la même signification que précédemment avec de l'acide sulfurique dans de l'acide fluorhydrique liquide sous une pression absolue de trifluorure de bore d'au moins un bar.

Le trifluorure de bore présente de grands avantages par rapport aux catalyseurs utilisés dans l'art antérieur. C'est un produit industriel, donc disponible sur le marché, et il se présente sous une forme gazeuse ce qui permet de le récupérer facilement et de le recycle en fin de réaction.

Les catalyseurs utilisés par Kozlov et ses collaborateurs tels que le pentafluorure d'antimoine sont liquides. De ce fait, ils ne sont récupérables et recyclables sans opération accessoire que lorsque les produits issus de la réaction sont gazeux aux températures de réaction ce qui n'est pas le cas dans la présente invention.

Selon un mode de réalisation préféré de l'invention dans la formule (I) R est l'hydrogène et Ar représente un radical monocyclique. Le dérivé aromatique est alors représenté par un halogénobenzène, un perhalogénoalkoxybenzène ou un perhalogénoalkylthiobenzène.

Encore plus préférentiellement, le dérivé benzénique est choisi parmi les halogénobenzènes.

L'acide sulfurique utilisé est l'acide sulfurique concentré du commerce. Il peut être remplacé sans sortir du cadre de l'invention par l'anhydride sulfurique ou un oleum et peut être présent au sein du milieu réactionnel sous la forme d'acide fluorosulfonique.

Avantageusement, le rapport molaire de l'acide sulfurique au composé aromatique est compris entre 0,4 et 1,5.

L'acide fluorhydrique utilisé est de préférence anhydre.

La mise en œuvre d'acide fluorhydrique aqueux entraînerait une consommation inutile de trifluorure de bore sous forme de HF, $BF_3$, $H_2O$ ($H_3O^+BF_4^-$).

Il est préférable de travailler avec une quantité de $BF_3$ telle que la pression absolue de $BF_3$ dans l'enceinte réactionnelle soit comprise entre 6 et 20 bars.

Plus la pression sera élevée, plus la vitesse de réaction augmentera. Une pression supérieure à 20 bars n'est pas exclue du domaine de l'invention mais n'apporte pas d'avantage particulier. L'homme de l'art adaptera donc la pression à l'économie du procédé.

Avantageusement, le rapport molaire de l'acide fluorhydrique au dérivé benzénique est compris entre 5 et 50. Encore plus préférentiellement, il est compris entre 10 et 30.

La température de la réaction est comprise de préférence entre 20 et 120 °C.

Les dérivés sulfonés obtenus dépendent des conditions de réaction ; ce sont soit des fluorures de sulfonyle répondant à la formule générale suivante :

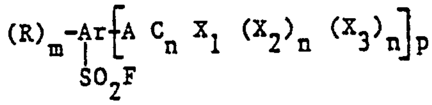

(II)

soit des diarylsulfones répondant à la formule générale suivante :

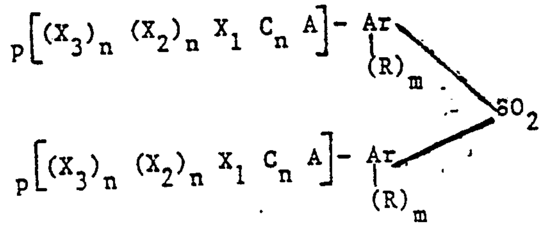

(III)

soit un mélange des deux.

La position de fixation du groupe $SO_2F$ sur le noyau se fera selon les règles de substitution bien connues de l'homme de l'art en fonction de la présence d'autres radicaux ortho, meta ou para orienteurs.

Dans les formules précédentes (II) et (III) n, m et p ont la signification précédente

et si n = 0 X a la signification précédente

si n = 1 $X_1$ $X_2$ et $X_3$ sont remplacés par du fluor

si n = 2 on obtient des dérivés de formule générale :

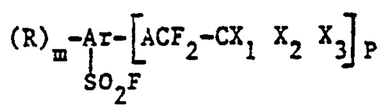

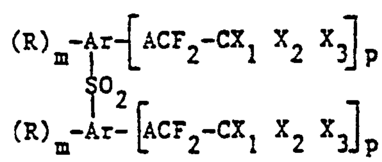

où $X_1$ $X_2$ $X_3$ ont la même signification que dans le produit de départ, l'échange avec les atomes de fluor ne se réalise que pour les halogènes situés en α de l'hétéroatome (A).

Le processus de la réaction se réalise en deux étapes.

Dans une première étape, il y a réaction entre l'acide sulfurique, le dérivé aromatique, l'HF et le $BF_3$ pour donner un fluorure de sulfonyle ; ce dernier peut réagir sur une deuxième molécule de dérivé aromatique pour donner une diarylsulfone. Suivant les conditions de réaction et les réactifs de départ, on réalise la première étape, les deux étapes ou seulement une partie de la deuxième.

Ainsi, dans le cas de composés pour lesquels p = 1 lorsque le rapport molaire acide sulfurique audit composé aromatique est inférieur ou égal à 0,5 la température comprise entre 80 et 120 °C et la durée de réaction d'au moins cinq heures, on obtiendra majoritairement des diphénylsulfones.

Dans le cas de composés pour lesquels p = 2 on obtiendra majoritairement des fluorures de sulfonyle.

Parmi les molécules répondant à la formule (I), on peut mettre en œuvre par exemple : le fluorobenzène, le chlorobenzène, le bromobenzène, l'iodobenzène, les difluorobenzènes, les dichlorobenzènes, les dibromobenzènes, les chlorofluorobenzènes, les chlorobromobenzènes, les bromofluorobenzènes, le trifluorométhoxybenzène, le trichlorométhoxybenzène, le tribromométhoxybenzène, le bromodichlorométhoxybenzène, le bromodifluorométhoxybenzène, les chlorotrifluorométhoxybenzènes, les fluorotrifluorométhoxybenzènes, le trifluorométhylthiobenzène, le trichlorométhylthiobenzène, les chlorotrifluorométhylthiobenzènes, les fluorotrifluorométhylthiobenzènes, le pentafluoroéthoxybenzène, le pentachloroéthoxybenzène, les fluorotoluènes, les chlorotoluènes, les fluorométhoxybenzènes, les chlorométhoxybenzènes, les fluorométhylthiobenzènes, les chlorophénols, les fluorophénols, les trifluorométhoxyphénols, les trifluorométhylthiophénols...

Parmi les fluorures de sulfonyle répondant à la formule (II) on peut citer : le fluorure de fluoro-4 benzénesulfonyle, le fluorure de chloro-4 benzénesulfonyle, le fluorure de bromo-4 benzènesulfonyle, le fluorure de trifluorométhoxy-4 benzène sulfonyle, le fluorure de trifluorométhylthio-4 benzène sulfonyle, le fluorure d'α,α-difluoro β,β,β-trichloroéthoxy-4 benzène sulfonyle, le fluorure de fluoro-2 méthyl-5 benzènesulfonyle, le fluorure de fluoro-3 méthyl-6 benzènesulfonyle, le fluorure de dichloro-3,4 benzènesulfonyle, le fluorure de dichloro-2,4 benzène sulfonyle, le fluorure de difluoro-2,4 benzènesulfonyle, le fluorure de fluoro-2 trifluorométhoxy-4 benzènesulfonyle, le fluorure de fluoro-4 trifluorométhoxy-2 benzènesulfonyle, le fluorure de fluoro-4 hydroxy-2 benzènesulfonyle, le fluorure de fluoro-4 méthoxy-2 benzènesulfonyle.

Parmi les diarylsulfones répondant à la formule (III), on peut citer : la difluoro-4,4' diphénylsulfone, la dichloro-4,4' diphénylsulfone, la dibromo-4,4' diphénylsulfone, la bis-trifluorométhoxy-4,4'-diphénylsulfone, la bis-trifluorométhylthio-4,4' diphénylsulfone, la bis (α,α-difluoro β,β,β-trichloroéthoxy)-4,4' diphénylsulfone, les difluorodiméthyldiphénylsulfones, les difluoro dihydroxy diphénylsulfones, les difluoro diméthoxy diphénylsulfones, la tétrafluoro-2,2',-4,4' diphénylsulfone, la tétrafluoro-3,3',-4,4' diphénylsulfone.

Les fluorures de sulfonyle ou les diarylsulfones obtenus par le procédé de l'invention sont utilisés comme intermédiaires de synthèse dans l'industrie pharmaceutique ou phytosanitaire (US 3970752 et J58067-604).

L'invention va être maintenant plus complètement décrite à l'aide des exemples qui vont suivre.

## Exemple 1

Dans un réacteur inox maintenu aux environs de 10 °C par un bain d'eau glacée, on introduit 100 g de HF anhydre, 32,4 g (0,2 m) de trifluorométhoxybenzène et 10 g (0,1 m) d'acide sulfurique concentré. On ferme le réacteur puis on introduit $BF_3$ gazeux jusqu'à la pression de 10 bars à 20 °C puis on laisse réagir 5 heures sous agitation. En fin de réaction, on refroidit le réacteur aux environs de 10 °C, on décomprime puis on coule le mélange réactionnel sur 200 g de glace pilée. On extrait alors par 3 fois 200 $cm^3$ de $CH_2Cl_2$. La phase organique est lavée par 2 fois 200 $cm^3$ d'eau permutée, 1 fois 200 $cm^3$ d'une solution de KOH à 5 % et 2 fois 200 $cm^3$ d'eau permutée puis séchée et évaporée. On recueille ainsi 33,7 g (rendement ≃ 87 %) de bis-trifluorométhox-4,4' diphénylsulfone. (Analyses infrarouge (IR), chromatographie phase gazeuse (CPG) et spectrométrie de masse F : 104,5 — 105 °C (MeOH)).

## Exemple 2

On procède d'une manière analogue à l'exemple 1 avec les composés et conditions suivantes :

| | |
|---|---|
| — Acide fluorhydrique : | 100 g (5 m) |
| — Fluorobenzène : | 19,2 g (0,2 m) |
| — $H_2SO_4$ concentré : | 10 g (0,1 m) |
| — $BF_3$ : | 7 bars à 20 °C |
| — Température : | 80 °C |
| — Durée : | 4 H 15 mn |

Par analyses IR, CPG et spectrométrie de masse, on note la composition suivante :

| | |
|---|---|
| — Fluorobenzène : | ≃ 30 % |
| — Fluorure de fluoro-4 benzènesulfonyle : | ≃ 20 % |
| — Difluoro-4,4' diphénylsulfone : | ≃ 50 % |

## Exemple 3

On procède d'une manière analogue à l'exemple 1 avec les composés et conditions suivantes :

| | |
|---|---|
| — Acide fluorhydrique : | 40 g (2 m) |
| — Chlorobenzène : | 22,5 g (0,2 m) |
| — $H_2SO_4$ concentré : | 10 g (0,1 m) |
| — $BF_3$ : | 8 bars à 20 °C |
| — Température : | 50 °C |
| — Durée : | 3 H 45 min |

Par analyses IR, CPG et spectrométrie de masse, on note la composition suivante :

| | |
|---|---|
| — Chlorobenzène : | ≃ 50 % |
| — Fluorure de chloro-4 benzènesulfonyle : | ≃ 20 % |
| — Dichloro-4,4′ diphénylsulfone | ≃ 30 % |

Exemple 4

On procède d'une manière analogue à l'exemple 1 avec les composés et conditions suivants :

| | |
|---|---|
| — Acide fluorhydrique : | 100 g (5 m) |
| — Bromobenzène : | 31,4 g (0,2 m) |
| — $H_2SO_4$ concentré : | 20 g (0,2 m) |
| — $BF_3$ : | 10 bars à 20 °C |
| — Température : | 50 °C |
| — Durée : | 4 H 40 min |

Par analyses IR, CPG et spectrométrie de masse, on note la composition suivante :

| | |
|---|---|
| — Bromobenzène : | 88 % |
| — Fluorure de bromo-4 benzènesulfonyle : | 10 % |
| — Dibromo-4,4′ diphénylsulfone : | 2 % |

Exemple 5

On procède d'une manière analogue à l'exemple 1 avec les composés et conditions suivants :

| | |
|---|---|
| — Acide fluorhydrique : | 120 g (6 m) |
| — Bromobenzène : | 31,4 g (0,2 m) |
| — $H_2SO_4$ concentré : | 10 g (0,1 m) |
| — $BF_3$ : | 6 bars |
| — Température : | 80 °C |
| — Durée : | 23 H 30 mn |

Par analyses IR, CPG et spectrométrie de masse, on note la composition suivante :

| | |
|---|---|
| — Fluorure de bromo-4 benzènesulfonyle : | ≃ 25 % |
| — Dibromo-4,4′ diphénylsulfone : | ≃ 50 % |

Exemple 6

On procède d'une manière analogue à l'exemple 1 avec les composés et conditions suivants :

| | |
|---|---|
| — Acide fluorhydrique : | 100 g (5 m) |
| — Fluoro-4 toluène : | 22 g (0,2 m) |
| — $H_2SO_4$ concentré : | 10 g (0,1 m) |
| — $BF_3$ : | 8 bars (20 °C) |
| — Température : | 34 °C |
| — Durée : | 5 heures |

Par analyses IR, CPG et spectrométrie de masse, on note la composition suivante :

| | |
|---|---|
| — Fluoro-4 toluène : | ≃ 60 % |
| — Fluorures de fluoro-2 méthyl-5 benzènesulfonyle et de fluoro-3 méthyl-6 benzènesulfonyle : | ≃ 35 % |
| — Difluorodiméthyldiphénylsulfones : | ≃ 5 % |

Exemple 7

On procède d'une manière analogue à l'exemple 1 avec les composés et conditions suivants :

| | |
|---|---|
| — Acide fluorhydrique : | 100 g (5 m) |
| — Fluorobenzène : | 9,6 g (0,1 m) |
| — $H_2SO_4$ concentré : | 14,7 g (0,15 m) |
| — $BF_3$ : | 10 bars à 20 °C |
| — Température : | 50 °C |
| — Durée : | 4 heures |

Par analyses IR, CPG et spectrométrie de masse, on note la composition suivante :

| | |
|---|---|
| — Fluorure de fluoro-4 benzènesulfonyle : | 40 % |
| — Difluoro-4,4' diphénylsulfone : | 50 % |
| — Divers : | 10 % |

Exemple 8

On procède d'une manière analogue à l'exemple 1 avec les composés et conditions suivants :

| | |
|---|---|
| — Acide fluorhydrique : | 60 g (3 m) |
| — Fluorobenzène : | 19,6 g (0,2 m) |
| — $H_2SO_4$ concentré : | 9,8 g (0,1 m) |
| — $BF_3$ : | 10 bars à 20 °C |
| — Température : | 23 °C |
| — Durée : | 19 heures |

Par analyses IR, CPG et spectrométrie de masse, on note la composition suivante :

| | |
|---|---|
| — Fluorure de fluoro-4 benzènesulfonyle : | 27 % |
| — Difluoro-4,4' diphénylsulfone : | 66 % |
| — Divers : | 7 % |

Exemple 9

On procède d'une manière analogue à l'exemple 1 avec les composés et conditions suivants :

| | |
|---|---|
| — Acide fluorhydrique : | 100 g (5 m) |
| — Fluorobenzène : | 19,6 g (0,2 m) |
| — $H_2SO_4$ concentré : | 9,8 g (0,1 m) |
| — $BF_3$ : | 10 bars à 20 °C |
| — Température : | 80 °C |
| — Durée : | 1 heure |

Par analyses IR, CPG et spectrométrie de masse, on note la composition suivante :

| | |
|---|---|
| — Fluorure de fluoro-4 benzènesulfonyle : | 48 % |
| — Difluoro-4,4' diphénylsulfone : | 48 % |
| — Divers : | 4 % |

**Revendications**

1. Procédé de préparation de diarylsulfones de formule (III)

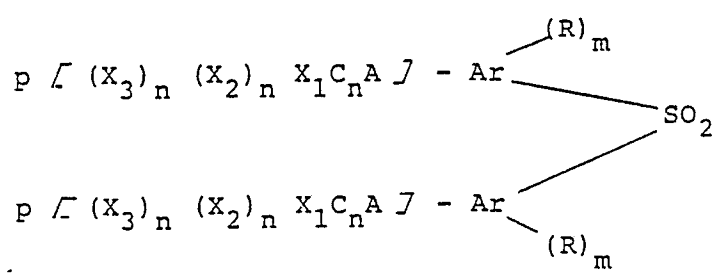

(III)

dans laquelle :

Ar représente un radical aromatique mono ou polycyclique

A représente une liaison covalente, un atome d'oxygène ou de soufre

$X_1$, $X_2$ et $X_3$ représentent chacun un atome d'halogène identique ou différent

n est compris entre 0 et 2 et quand A est une liaison covalente

n est égal à 0

p est égal à 1 ou 2

R est choisi parmi l'hydrogène, alkyle de C1 à C6, alkoxy, alkylthio, hydroxyle

m est égal à 1

caractérisé par le fait que l'on fait réagir un composé de formule $(R)_m — Ar — [AC_n X_1(X_2)_n (X_3)_n]_p$ dans laquelle R, Ar, A, $X_1$, $X_2$, $X_3$, m, n, p ont la même signification que précédemment avec de l'acide sulfurique dans de l'acide fluorhydrique liquide sous une pression absolue de trifluorure de bore d'au moins un bar, à une température inférieure à 120 °C.

2. Procédé selon la revendication 1 caractérisé par le fait que R est l'hydrogène.

3. Procédé selon la revendication 1 caractérisé par le fait que Ar est un radical aromatique monocyclique.

4. Procédé selon l'une des revendications précédentes caractérisé par le fait que A est une liaison covalente et n est égal à 0.

5. Procédé selon l'une quelconque des revendications précédentes caractérisé par le fait que le dérivé aromatique est le fluorobenzène.

6. Procédé selon la revendication 1 caractérisé par le fait que le rapport molaire acide fluorhydrique au dérivé aromatique est compris entre 5 et 50.

7. Procédé selon la revendication 1 caractérisé par le fait que le rapport molaire de l'acide sulfurique au dérivé aromatique est compris entre 0,4 et 1,5.

8. Procédé selon l'une quelconque des revendications caractérisé par le fait que l'acide fluorhydrique mis en œuvre est de l'acide fluorhydrique anhydre.

9. Procédé selon l'une quelconque des revendications précédentes caractérisé par le fait qu'on utilise une quantité de trifluorure de bore telle que la pression absolue de $BF_3$ dans l'enceinte réactionnelle soit comprise entre 6 et 20 bars.

10. Procédé selon l'une quelconque des revendications précédentes caractérisé par le fait que la réaction est réalisée à une température comprise entre 20 et 120 °C.

## Claims

1. Process for the preparation of diaryl sulphones of formula (III)

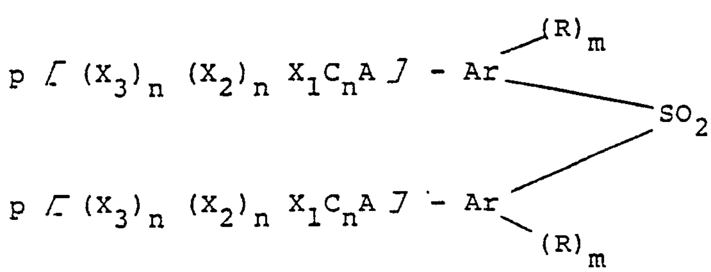

(III)

in which :

Ar denotes a mono- or polycyclic aromatic radical

A denotes a covalent bond or an oxygen or sulphur atom each of $X_1$, $X_2$ and $X_3$ denotes an identical or different halogen atom

n is between 0 and 2 and when A is a covalent bond n is equal to 0

p is equal to 1 or 2

R is chosen from hydrogen, $C_1$-$C_6$ alkyl, alkoxy, alkylthio and hydroxyl

m is equal to 1

characterized in that a compound of formula $(R)_m — Ar — [AC_n X_1(X_2)_n (X_3)_n]$ p in which R, Ar, A, $X_1$, $X_2$, $X_3$, m, n and p have the same meaning as above, is reacted with sulphuric acid in liquid hydrofluoric acid at an absolute pressure of borontrifluoride of at least one bar, at a temperature below 120 °C.

2. Process according to Claim 1, characterized in that R is hydrogen.

3. Process according to Claim 1, characterized in that Ar is a monocyclic aromatic radical.

4. Process according to one of the preceding claims, characterized in that A is a covalent bond and n is equal to 0.

5. Process according to any one of the preceding claims, characterized in that the aromatic derivative is fluorobenzene.

6. Process according to Claim 1, characterized in that the molar ratio of hydrofluoric acid to the aromatic derivative is between 5 and 50.

7. Process according to Claim 1, characterized in that the molar ratio of sulphuric acid to the aromatic derivative is between 0.4 and 1.5.

8. Process according to any one of the preceding claims, characterized in that the hydrofluoric acid employed is anhydrous hydrofluoric acid.

9. Process according to any one of the preceding claims, characterized in that a quantity of borontrifluoride is employed, such that the absolute pressure of $BF_3$ in the reaction enclosure is between 6 and 20 bars.

10. Process according to any one of the preceding claims, characterized in that the reaction is carried out at a temperature of between 20 and 120 °C.

**Patentansprüche**

1. Verfahren zur Herstellung von Diarylsulfonen der Formel (III)

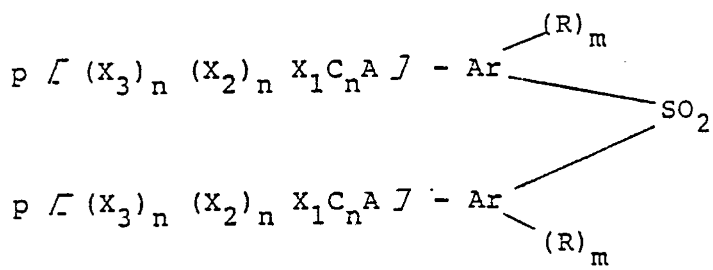

(III)

in der:

Ar einen aromatischen mono- oder polycyclischen Rest darstellt

A eine covalente Bindung darstellt, ein Sauerstoffatom oder ein Schwefelatom

$X_1$, $X_2$ und $X_3$ jeweils ein identisches oder unterschiedliche Halogenatome darstellen

n zwischen 0 und 2 liegt und wenn A eine kovalente Bindung ist gleich 0 ist

p gleich 1 oder 2 ist

R ausgewählt wird aus Wasserstoff, Alkyl mit C1 bis C6, Alkoxy, Alkylthio, Hydroxyl

m gleich 1 ist

dadurch gekennzeichnet, daß man eine Verbindung der Formel $(R)_m$—Ar—$[AC_n X_1(X_2)_n (X_3)_n]$ p in der R, Ar, A, $X_1$, $X_2$, $X_3$, m, n und p die gleiche Bedeutung wie oben haben mit Schwefelsäure in flüssiger Fluorwasserstoffsäure bei einem absoluten Druck an Bortrifluorid von wenigstens einem Bar und bei einer Temperatur unterhalb von 120 °C reagieren läßt.

2. Verfahren nach Anspruch 1, dadurch gekennzeichnet, daß R Wasserstoff ist.

3. Verfahren nach Anspruch 1, dadurch gekennzeichnet, daß Ar ein monocyclischer aromatischer Rest ist.

4. Verfahren nach einem der vorhergehenden Ansprüche, dadurch gekennzeichnet, daß A eine covalente Bindung ist und n gleich 0 ist.

5. Verfahren nach einem der vorhergehenden Ansprüche, dadurch gekennzeichnet, daß die aromatische Verbindung Fluorbenzol ist.

6. Verfahren nach Anspruch 1, dadurch gekennzeichnet, daß das molare Verhältnis Fluorwasserstoffsäure zu aromatischer Verbindung zwischen 5 und 50 beträgt.

7. Verfahren nach Anspruch 1, dadurch gekennzeichnet, daß das molare Verhältnis von Schwefelsäure zu aromatischer Verbindung zwischen 0,4 und 1,5 beträgt.

8. Verfahren nach einem der Ansprüche, dadurch gekennzeichnet, daß die Fluorwasserstoffsäure, die umgesetzt wird, wasserfreie Fluorwasserstoffsäure ist.

9. Verfahren nach einem der vorhergehenden Ansprüche, dadurch gekennzeichnet, daß man eine solche Menge an Bortrifluorid verwendet, daß der absolute Druck an $BF_3$ im Reaktionsgefäß zwischen 6 und 20 bar beträgt.

10. Verfahren nach einem der vorhergehenden Ansprüche, dadurch gekennzeichnet, daß die Reaktion bei einer Temperatur zwischen 20 und 120 °C durchgeführt wird.